# EUROPEAN PATENT SPECIFICATION

(11) **EP 2 389 367 B1**
(45) Date of publication and mention of the grant of the patent: **18.07.2018**
(21) Application number: 10701427.6
(22) Date of filing: 22.01.2010
(51) Int. Cl.: C07D 257/06, C06C 7/00, C06B 41/00

(54) **PREPARATION OF A LEAD-FREE PRIMARY EXPLOSIVE**
HERSTELLUNG EINES BLEIFREIEN PRIMÄREXPLOSIVSTOFFS
PRÉPARATION D'UN EXPLOSIF PRIMAIRE SANS PLOMB

(30) Priority: 23.01.2009 US 146700 P
(43) Date of publication of application: 30.11.2011
(73) Proprietor: Pacific Scientific Energetic Materials Company, Chandler, AZ 85226 (US)
(72) Inventor: FRONABARGER, John, W., Sun Lakes, AZ 85248 (US); WILLIAMS, Michael, D., Gilbert, AZ 85233 (US)
(74) Representative: Brann AB
(86) International application number: PCT/US2010/021695
(87) International publication number: WO 2010/085583

(56) References cited:
- WO-A2-2008/048351
- US-A- 2 066 954
- US-A- 4 093 623
- TALAWAR ET AL: "Energetic co-ordination compounds: synthesis, characterization and thermolysis studies on bis-(5-nitro-2H-tetrazolato-N<2>)tetraammi ne cobalt(III) perchlorate (BNCP) and its new transition metal (Ni/Cu/Zn) perchlorate analogues" JOURNAL OF HAZARDOUS MATERIALS, ELSEVIER, AMSTERDAM, NL, vol. 120, no. 1-3, 5 April 2005 (2005-04-05), pages 25-35, XP022384449 ISSN: 0304-3894
- "MERCK INDEX 13th", 1 January 1983 (1983-01-01)

## Description

### CROSS-REFERENCE TO RELATED APPLICATIONS

### FIELD OF THE INVENTION

This invention relates to explosives, and in particular to preparation of a primary explosive that is free of lead.

### BACKGROUND OF THE INVENTION

Explosive materials have a wide variety of applications. Primary explosives are sensitive explosive materials that are used, in relatively small quantities, to initiate a secondary or main explosive charge. Primary explosives should be sufficiently sensitive to be detonated reliably but not so sensitive as to be exceedingly dangerous to handle. Moreover, primary explosives should have sufficient thermal stability so as to not decompose on extended storage or temperature fluctuation, Many primary explosives in current use contain lead, with the most well-known example being lead azide. These lead-containing explosives are undesirable from an environmental standpoint, since their use and manufacture can contribute to or cause lead contamination.

Thus, there is a need in the art for lead-free explosive materials and in particular for lead-free primary explosives. Certain lead-free primary explosives have been proposed. For instance, nitrotetrazole-based primary explosives have been proposed in U.S. Pat. Nos. 4,093,623 and 4,094,879, as well as in U.S. Patent App. Pub. No. 2006/0030715. For a variety of reasons, some of these proposed compounds have failed to serve as commercially viable substitutes for lead-containing primary explosives, while others exhibit characteristics that make them undesirable for at least some commercial applications. For example, U.S. Patent App. Pub. No. 2006/0030715 discloses certain nitrotetrazole complexes (including copper(II) complexes) which form a crystalline structure that is difficult to work with from a handling and ordinance loading standpoint.

Also the published international patent application WO 2008/048351 A2 discloses a compound and material that may be used as a lead-free primary explosive as well as a method for preparing lead-free primary explosives.

### SUMMARY OF THE INVENTION

Embodiments of the invention are directed to a compound and material that may be used as a lead-free primary explosive, and methods for preparing such compound and material.

In one embodiment, a compound is prepared by the reaction of cupric chloride, sodium 5-nitrotetrazolate, sodium ascorbate, and water.

In another embodiment, a method of preparing a compound suitable as a primary explosive comprises the steps of: providing a cupric salt; providing a solvent; providing a 5-nitrotetrazolate salt; combining the cupric salt, solvent and 5-nitrotetrazolate salt to form a mixture; heating the mixture; adding a reducing agent; and heating the combination.

In some embodiments, a compound is prepared by providing cupric chloride; providing water; providing sodium 5-nitrotetrazolate; combining the cupric chloride, water, and sodium 5-nitrotetrazolate to form a mixture; heating the mixture; adding a solution of sodium ascorbate; and heating the combination.

### BRIEF DESCRIPTION OF SEVERAL VIEWS OF THE DRAWINGS

FIG. 1 shows the results of a differential scanning calorimetry (DSC) analysis on a material prepared according to the present techniques,
FIG. 2 shows the results of a Fourier Transform Infrared Spectroscopy (FTIR) analysis on a material prepared according to the present techniques.

### DETAILED DESCRIPTION OF THE INVENTION

One aspect of the present subject matter is preparation of the compound copper(I) nitrotetrazolate. Also contemplated is any mixture which contains copper(I) nitrotetrazolate in a significant quantity (e.g. greater than about 1 weight percent, or alternatively, greater than about 5 weight percent).

Methods for preparing copper(I) nitrotetrazolate are contemplated in the present application. Copper(I) nitrotetrazolate may be prepared by reacting a copper(II) salt (for example, cupric chloride), a 5-nitrotetrazolate salt (for example, sodium 5-nitrotetrazolate) and a reducing agent (for example sodium ascorbate) in a solvent (for example, water). Any suitable copper(II) salt, or combination of copper(II) salts, may be employed. Suitable copper(II) salts include, but are not limited to, cupric chloride and cupric bromide. Likewise, any suitable 5-nitrotetrazolate salt, or combination of 5-nitrotetrazolate salts, may be employed. Suitable 5-nitrotetrazolate salts include, but are not limited to, sodium 5-nitrotetrazolate and potassium 5-nitrotetrazolate. Likewise, any suitable reducing agent, or combination of reducing agents, may be employed. Suitable reducing agents include, but are not limited to, sodium ascorbate and ascorbic acid. Likewise, any suitable solvent, or combination of solvents, may be employed. Suitable solvents include, but are not limited to, water, dimethyl sulfoxide (DMSO), as well as other polar organic solvents.

It will be understood that ionic versions of the salts referred to above may be employed in the preparation of copper(I) nitrotetrazolate. In other words, copper(I) nitrotetrazolate may be prepared by a reaction in which copper(I) ions and 5-nitrotetrazolate ions are combined to form copper(I) nitrotetrazolate.

The components may be reacted under conditions suitable to synthesize copper(I) nitrotetrazolate. Alternatively, the components may be reacted by mixing them together and then heating the mixture. The mixture may be heated in the temperature range of 70°C to 150°C, alternatively in the temperature range of 80°C to 130°C, alternatively to 100°C. As yet another alternative, a reflux condenser may be employed, and the mixture may be heated to the reflux point. The duration of the heating or refluxing step may be a duration that is greater than about 1 minute, alternatively greater than about 35 minutes, alternatively from about 20 minutes to about 2 hours, alternatively from about 35 minutes to about 1 hour, alternatively about 50 minutes.

Regarding quantities of the components employed, 5-nitrotetrazolate may be supplied in a molar ratio of about 0.5 moles to about 2 moles 5-nitrotetrazolate per mole of copper(II). Alternatively, 5-nitrotetrazolate may be supplied in a molar ratio of about 0.8 moles to about 1.5 moles 5-nitrotetrazolate per mole of copper(II). Alternatively, 5-nitrotetrazolate may be supplied in a molar ratio of about 1 mole to about 1.2 moles 5-nitrotetrazolate per mole of copper(II), For example, sodium 5-nitrotetrazolate (NaNT) may be supplied in a molar ratio of about 0.5 moles to about 4 moles NaNT per mole of cupric chloride, alternatively about 0.8 moles to about 1.5 moles NaNT per mole of cupric chloride, alternatively about 1 mole to about 1.2 moles NaNT per mole of cupric chloride. Similarly, the reducing agent may be supplied in a molar ratio of about 0.5 mole to about 2 moles per mole of copper (II). Alternatively, the reducing agent may be supplied in a molar ratio of about 0.8 moles to about Alternatively, the product may be washed in multiple steps and in any order with both water and alcohol. For example, the product may be washed sequentially with water and then isopropanol. The product may then be dried. For example, the product may be air dried. Alternatively the product may be dried in an oven at 65 to 80°C.

The present application also contemplates products made by the methods described above. In other words, the present application contemplates products made by reacting cupric salt (for example, cupric chloride), 5-nitrotetrazolate salt (for example, sodium 5-nitrotetrazolate), and a reducing agent (for example sodium ascorbate) in water, under the conditions and component quantities described above.

The products contemplated and made by the methods of the present application (in at least some aspects of the present subject matter, copper(I) nitrotetrazolate) are free of lead and have been found suitable for use as explosives and, in particular, as primary explosives. Thus, the present application also contemplates methods for preparing compounds suitable for use as primary explosives, and explosive devices employing such compounds. Benefits include low cost, ease of preparation and low toxicity waste streams and health benefits associated with low lead materials in both military and commercial applications.

The products contemplated and made by the methods of the present application (including copper(I) nitrotetrazolate) exhibit a crystalline structure that is suitable for loading and handling,

The foregoing is provided for purposes of illustrating, explaining, and describing embodiments of the present invention. Further modifications and adaptations to these embodiments will be apparent to those skilled in the art and may be made without departing from the scope or spirit of the invention.

### EXAMPLES

The following examples demonstrate the preparation and characterization of a material as taught herein.

### Example 1

Copper(I) nitrotetrazolate was prepared as follows. To 100mL of a stirred hot (95-100°C) aqueous solution of copper(II) chloride (0.79g, 0.008 mol) and sodium 5-nitrotetrazolate dihydrate (1.70g, 0.0096 mol) in a 250mL beaker was added 8mL of a 0.5 molar aqueous solution of sodium ascorbate at a rate of 1 mL/minute using a syringe pump. After the eight minute addition, the reaction mixture was boiled for an additional two 1.5 moles per mole of copper (II). Alternatively, the reducing agent may be supplied in a molar ratio of about 1 mole to about 1.2 moles per mole of copper (II). For example, sodium ascorbate may be supplied in a molar ratio of about 0.5 moles to about 4 moles per mole of cupric chloride, alternatively about 0.8 moles to about 1.5 moles per mole of cupric chloride, alternatively about 1 mole to about 1.2 moles per mole of cupric chloride.

A solvent may be supplied in an amount that is suitable to effectuate the reaction between 5-nitrotetrazolate and formed copper(I). For example, water (or other solvent) may be supplied in an amount that is suitable to effectuate the reaction between a 5-nitrotetrazolate salt and a copper(I) salt. As a more specific example, water (or other solvent) may be supplied in an amount that is suitable to effectuate the reaction between NaNT and formed cuprous chloride.

The reaction components may be combined in any order or sequence suitable to effectuate the reaction. By way of non-limiting example, the reaction of 5-nitrotetrazolate salt and formed copper(I) salt may be carried out by adding an aqueous solution of 5-nitrotetrazolate salt to an aqueous suspension of copper(II) salt and adding a suitable reducing agent, or vice versa.

The copper(I) nitrotetrazolate formed by the reaction of cupric salt (for example, cupric chloride), a reducing agent (for example sodium ascorbate), water and 5-nitrotetrazolate salt (for example, sodium 5-nitrotetrazolate) may be .a precipitate. The precipitate may be separated by a suitable method known to those of skill in the art. Alternatively, the precipitate may be separated by filtration. As yet another alternative, the precipitate may be separated using a flotation technique. It may be desirable to separate finer or lighter precipitate particles from coarser or heavier precipitate particles (for example, the coarser or heavier particles may be desirable from the standpoint of easy handling and loading). A flotation technique may be employed to achieve such a separation, as may other techniques known to those of skill in the art. Alternatively, the fine particles may be removed by careful decanting. Alternatively, the precipitate (which may, for example, be a dark brown precipitate) is collected over filter paper.

The precipitate formed by the reaction of cupric salt (for example, cupric chloride), a reducing agent (for example sodium ascorbate), water and 5-nitrotetrazolate salt (for example, sodium 5-nitrotetrazolate) may be washed. For example, the product may be washed either a single time or multiple times with water. Alternatively, the product may be washed either a single time or multiple times with alcohol, for example, isopropanol. minutes. The precipitate that was formed was collected on Whatman No. 1 filter paper, washed three times with water, twice with isopropanol, and then dried in a convection oven at 70°C. The yield of small red rust crystals was 1.218g (85.7%).

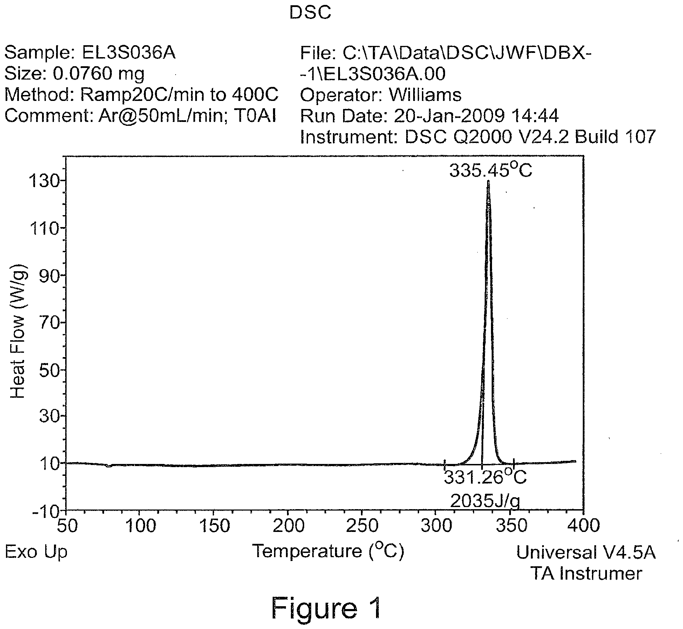

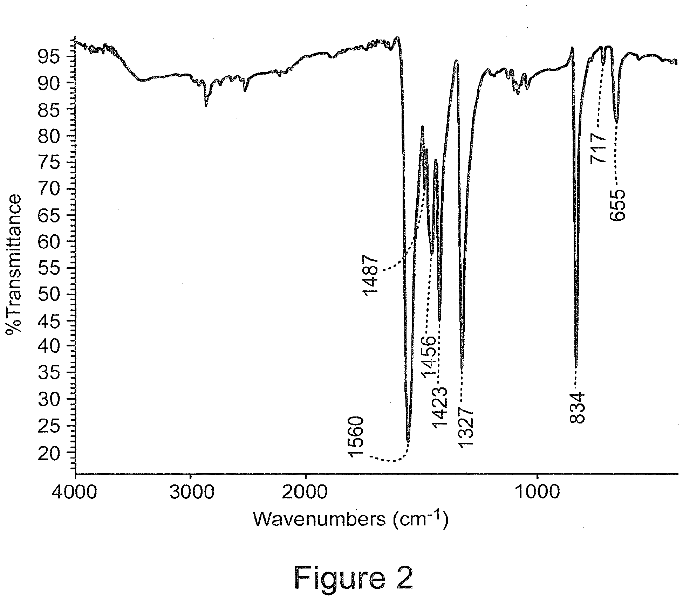

The results of a differential scanning calorimetry (DSC) analysis on the solid are shown in FIG. 1. The results of a Fourier Transform Infrared Spectroscopy (FTIR) analysis on the solid are shown in FIG. 2.

### Example 2

Copper(I) nitrotetrazolate was prepared as follows. To 100mL of a stirred hot (95-100°C) aqueous solution of copper(II) chloride (0.79g) and sodium 5-nitrotetrazolate dehydrate (1.70g) in a 250mL beaker was added 8mL of a 0.5 molar aqueous solution of ascorbic acid at a rate of 1 mL/minute using a syringe pump. After the eight minute addition, the reaction mixture was boiled for an additional two minutes. The precipitate that formed was collected on Whatman No. 1 filter paper, washed three times with water, twice with isopropanol, and then dried in a convection oven at 70°C.

While the present subject matter has been described and illustrated by reference to particular embodiments, it will be appreciated by those of ordinary skill in the art that the subject matter lends itself to many different variations not illustrated herein. For these reasons, then, reference should be made solely to the appended claims for purposes of determining the true scope of the present invention.

## Claims

1. A method for preparing a compound suitable for use as a primary explosive, comprising the steps of:
(a) providing a cupric salt;
(b) providing a solvent;
(c) providing a 5-nitrotetrazolate salt;
(d) combining the cupric salt, the solvent, and the 5-nitrotetrazolate salt to form a mixture;
(e) heating the mixture to a temperature of 80 - 130 °C;
(f) providing a reducing agent to form a combination containing a cuprous salt; and
(g) heating the combination.

2. The method of claim 1, wherein the cupric salt is selected from the group consisting of cupric chloride and cupric bromide.

3. The method of claim 1, wherein the solvent is a polar organic solvent.

4. The method of claim 1, wherein the solvent is selected from the group consisting of water and dimethyl sulfoxide.

5. The method of claim 1, wherein the 5-nitrotetrazolate salt is selected from the group consisting of sodium 5-nitrotetrazolate and potassium 5-nitrotetrazolate.

6. The method of claim 1, wherein the reducing agent is selected from the group consisting of sodium ascorbate and ascorbic acid.

7. The method of claim 1, wherein the 5-nitrotetrazolate salt is provided in an amount of about 0.5 moles to about 5 moles per mole of cupric salt.

8. The method of claim 1, wherein the reducing agent is provided in an amount of about 0.5 moles to about 4 moles per mole of cupric salt.

9. The method of claim 1, wherein the 5-nitrotetrazolate salt is provided in a molar ratio of about 1 mole per mole of cupric salt.

10. The method of claim 1, wherein the mixture is heated to 100 °C.

11. A product prepared by a process comprising the steps of:
(a) providing cupric chloride;;
(b) providing water;
(c) providing sodium 5-nitrotetrazolate;
(d) combining the cupric chloride, the water, and the sodium 5-nitrotetrazolate to form a mixture;
(e) heating the mixture;
(f) adding a solution of sodium ascorbate; and
(g) heating the combination.

12. The product of claim 11, **characterized by** a Differential Scanning Calorimetry curve with a distinguishing peak at about 335 °C.

13. The product of claim 11, **characterized by** a Differential Scanning Calorimetry curve substantially as shown in Fig. 1.

14. The product of claim 11, **characterized by** a Fourier Transform Infrared Spectroscopy spectra with peaks at about 1560, 1487, 1456, 1423 and 1327.

15. The product of claim 11, **characterized by** a Fourier Transform Infrared Spectroscopy spectrum as shown in Fig. 2.

16. A reaction product of:
(a) cupric chloride;
(b) sodium 5-nitrotetrazolate;
(c) sodium ascorbate; and
(d) water.

## Patentansprüche

1. Verfahren zur Herstellung einer zur Verwendung als ein Primärexplosivstoff geeigneten Verbindung, umfassend die Schritte:
(a) Bereitstellen eines Kupfer(II)salzes;
(b) Bereitstellen eines Lösungsmittels;
(c) Bereitstellen eines 5-Nitrotetrazolatsalzes;
(d) Vereinigen des Kupfer(II)salzes, des Lösungsmittels und des 5-Nitrotetrazolatsalzes zur Bildung eines Gemisches;
(e) Erhitzen des Gemisches auf eine Temperatur von 80-130°C;
(f) Bereitstellen eines Reduktionsmittels zur Bildung einer ein Kupfer(I)salz enthaltenden Kombination; und
(g) Erhitzen der Kombination.

2. Verfahren nach Anspruch 1, wobei das Kupfer(II)salz ausgewählt ist aus der Gruppe, bestehend aus Kupfer(II)chlorid und Kupfer(II)bromid.

3. Verfahren nach Anspruch 1, wobei das Lösungsmittel ein polares organisches Lösungsmittel ist.

4. Verfahren nach Anspruch 1, wobei das Lösungsmittel ausgewählt ist aus der Gruppe, bestehend aus Wasser und Dimethylsulfoxid.

5. Verfahren nach Anspruch 1, wobei das 5-Nitrotetrazolatsalz ausgewählt ist aus der Gruppe, bestehend aus Natrium-5-nitrotetrazolat und Kalium-5-nitrotetrazolat.

6. Verfahren nach Anspruch 1, wobei das Reduktionsmittel ausgewählt ist aus der Gruppe, bestehend aus Natriumascorbat und Ascorbinsäure.

7. Verfahren nach Anspruch 1, wobei das 5-Nitrotetrazolatsalz in einer Menge von etwa 0,5 Mol bis etwa 5 Mol pro Mol Kupfer(II)salz bereitgestellt wird.

8. Verfahren nach Anspruch 1, wobei das Reduktionsmittel in einer Menge von etwa 0,5 Mol bis etwa 4 Mol pro Mol Kupfer(II)salz bereitgestellt wird.

9. Verfahren nach Anspruch 1, wobei das 5-Nitrotetrazolatsalz in einem Molverhältnis von etwa 1 Mol pro Mol Kupfer(II)salz bereitgestellt wird.

10. Verfahren nach Anspruch 1, wobei das Gemisch auf 100°C erhitzt wird.

11. Produkt, hergestellt durch ein Verfahren, umfassend die Schritte:
(a) Bereitstellen von Kupfer(II)chlorid;
(b) Bereitstellen von Wasser;
(c) Bereitstellen von Natrium-5-nitrotetrazolat;
(d) Vereinigen des Kupfer(II)chlorids, des Wassers und des Natrium-5-nitrotetrazolats zur Bildung eines Gemisches;
(e) Erhitzen des Gemisches;
(f) Zusetzen einer Lösung von Natriumascorbat; und
(g) Erhitzen der Kombination.

12. Produkt nach Anspruch 11, **gekennzeichnet durch** eine Dynamische Differenzkalorimetriekurve mit einem charakteristischen Peak bei etwa 335°C.

13. Produkt nach Anspruch 11, **gekennzeichnet durch** eine wie im Wesentlichen in Fig. 1 gezeigte Dynamische Differenzkalorimetriekurve.

14. Produkt nach Anspruch 11, **gekennzeichnet durch** ein Fourier-Transformations-Infrarot-Spektroskopie-Spektrum mit Peaks bei etwa 1560, 1487, 1456, 1423 und 1327.

15. Produkt nach Anspruch 11, **gekennzeichnet durch** ein wie in Fig. 2 gezeigtes Fourier-Transformations-Infrarot-Spektroskopie-Spektrum.

16. Reaktionsprodukt von:
(a) Kupfer(II)chlorid;
(b) Natrium-5-nitrotetrazolat;
(c) Natriumascorbat; und
(d) Wasser.

## Revendications

1. Procédé de préparation d'un composé utilisable comme explosif primaire, comprenant les étapes de :
(a) fournir un sel cuivrique ;
(b) fournir un solvant ;
(c) fournir un sel de 5-nitrotétrazolate ;
(d) combiner le sel cuivrique, le solvant et le sel de 5-nitrotétrazolate pour former un mélange ;
(e) chauffer le mélange à une température de 80 à 130 °C ;
(f) fournir un agent réducteur pour former une combinaison contenant un sel cuivreux ; et
(g) chauffer la combinaison.

2. Procédé selon la revendication 1, dans lequel le sel cuivrique est choisi dans le groupe constitué par le chlorure cuivrique et le bromure cuprique.

3. Procédé selon la revendication 1, dans lequel le solvant est un solvant organique polaire.

4. Procédé selon la revendication 1, dans lequel le solvant est choisi dans le groupe constitué par l'eau et le diméthyl sulfoxyde.

5. Procédé selon la revendication 1, dans lequel le sel de 5-nitrotétrazolate est choisi dans le groupe constitué par le 5-nitrotétrazolate de sodium et le 5-nitrotétrazolate de potassium.

6. Procédé selon la revendication 1, dans lequel l'agent réducteur est choisi dans le groupe constitué par l'ascorbate de sodium et l'acide ascorbique.

7. Procédé selon la revendication 1, dans lequel le sel de 5-nitrotétrazolate est fourni en une quantité d'environ 0,5 mole à environ 5 moles par mole de sel cuivrique.

8. Procédé selon la revendication 1, dans lequel l'agent réducteur est fourni en une quantité d'environ 0,5 mole à environ 4 moles par mole de sel cuivrique.

9. Procédé selon la revendication 1, dans lequel le sel de 5-nitrotétrazolate est fourni dans un rapport molaire d'environ 1 mole par mole de sel cuivrique.

10. Procédé selon la revendication 1, dans lequel le mélange est chauffé à 100 °C.

11. Produit préparé par un procédé comprenant les étapes de :
(a) fournir du chlorure cuivrique ;
(b) fournir de l'eau ;
(c) fournir du 5-nitrotétrazolate de sodium ;
(d) combiner le chlorure cuivrique, l'eau et le 5-nitrotétrazolate de sodium pour former un mélange ;
(e) chauffer le mélange ;
(f) ajouter une solution d'ascorbate de sodium ; et
(g) chauffer la combinaison.

12. Produit selon la revendication 11, **caractérisé par** une courbe de calorimétrie à balayage différentiel avec un pic distinctif à environ 335 °C.

13. Produit selon la revendication 11, **caractérisé par** une courbe de calorimétrie à balayage différentiel sensiblement comme représenté sur la figure 1.

14. Produit selon la revendication 11, **caractérisé par** un spectre de spectroscopie infrarouge à transformée de Fourier avec des pics à environ 1560, 1487, 1456, 1423 et 1327.

15. Produit selon la revendication 11, **caractérisé par** un spectre de spectroscopie infrarouge à transformée de Fourier tel que représenté sur la figure 2.

16. Produit réactionnel :
(a) de chlorure cuivrique ;
(b) de 5-nitrotétrazolate de sodium ;
(c) d'ascorbate de sodium ; et
(d) d'eau.
